(19)

**Europäisches Patentamt**
**European Patent Office**
**Office européen des brevets**

(11)  **EP 4 083 185 A1**

(12)  # EUROPEAN PATENT APPLICATION

(43) Date of publication:
**02.11.2022  Bulletin 2022/44**

(21) Application number: **22164604.5**

(22) Date of filing: **28.03.2022**

(51) International Patent Classification (IPC):
$C12M\ 1/34^{(2006.01)}$   $C12M\ 1/36^{(2006.01)}$
$C12N\ 1/20^{(2006.01)}$   $C07K\ 14/195^{(2006.01)}$

(52) Cooperative Patent Classification (CPC):
**C12M 41/48; C07K 14/195; C12M 41/32;**
**C12M 41/34; C12N 1/20**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **02.04.2021  LT 2021514**

(71) Applicant: **Kaunas University of Technology**
**44249 Kaunas (LT)**

(72) Inventors:
• **Galvanauskas, Vytautas**
  **53107 Kaunas (LT)**
• **Simutis, Rimvydas**
  **46307 Kaunas (LT)**

• **Levisauskas, Donatas**
  **51390 Kaunas (LT)**
• **Urniezius, Renaldas**
  **53331 Kaunas (LT)**
• **Vaitkus, Vygandas**
  **45462 Kaunas (LT)**
• **Tekorius, Tomas**
  **49327 Kaunas (LT)**
• **Butkus, Mantas**
  **47222 Kaunas (LT)**
• **Survila, Arnas**
  **50393 Kaunas (LT)**

(74) Representative: **Klimaitiene, Otilija**
**AAA Law**
**P.O.Box 33**
**A. Gostauto street 40B**
**03163 Vilnius (LT)**

(54)  **METHOD FOR DESIGN AND ADAPTATION OF FEEDING PROFILES FOR RECOMBINANT E.COLI FED-BATCH CULTIVATION PROCESSES**

(57)    The invention discloses a method of supplying a feeding substrate to a bioreactor. Specifically, the method comprises steps of developing and adapting robust feeding profiles based on the analysis of experimental data to rationally manage fed-batch recombinant E.coli cultivation processes, to ensure the stability, repeatability, and high productivity of these processes. The method is based on the regulation of glucose uptake in culture medium using well-prepared robust glucose feeding algorithms, which are formed based on data from primary culture experiments and specialized experiments with recombinant *E.coli* bacteria. The robustness of the supplied glucose feeding profiles is ensured by the formation of appropriate glucose uptake rate limitation levels during the cultivation of *E.coli* bacteria. The adaptation of the profiles is performed in real-time, using a specialized, software sensor-based indicator that signals the excess glucose concentration accumulating in the bioreactor medium. The indicator values are evaluated by calculating the ratio of the amount of oxygen consumed to the amount of glucose supplied to the bioreactor during the selected culture time interval. The indicator values obtained are compared with the indicator values determined for the reference process, which ensures adequate ro- bustness of the process and a high amount of the target recombinant protein to be synthesized. Based on the obtained deviations, the reference glucose feeding profile is adapted in real-time by the controller.

Fig. 1

EP 4 083 185 A1

**Description**

FIELD OF INVENTION

The invention relates to automatic control technologies for bioreactors. More specifically, it discloses a method for automated control of the cultivation conditions in microorganism cultures, by selecting rational modes of supplying the feeding substrate into a bioreactor to ensure robust, highly efficient, and reproducible processes of recombinant *E.coli* fed-batch cultivation.

BACKGROUND ART

Much of the recombinant proteins used for biomedical purposes are currently produced by processes of recombinant *E.coli* fed-batch cultivation in special bioreactors. Due to the rapid growth and high-rate synthesis of the target product, *E.coli* bacteria are particularly useful for large-scale industrial production of recombinant proteins.

In traditional batch-cultivation processes, all the cultivation medium is supplied into the bioreactor once at the beginning of the cultivation process, and the cultivation processes are uncontrolled.

In modern advanced recombinant *E.coli* cultivation processes, the cultivation medium is supplied to the microorganisms gradually throughout the cultivation process (Fed-Batch Cultivation Processes). This method of cultivating bacteria allows the concentration of the substrate (mainly, glucose) in the cultivation medium to be controlled to avoid inhibition of bacterial growth rate due to high concentrations of the substrate and to limit the formation of by-products that reduce bacterial growth rate and target-product synthesis rate.

The rate of supply of the feeding substrate (mainly, glucose) to the bioreactor is a key factor in determining the controllability, robustness, reproducibility, and rate of synthesis of the target recombinant protein in recombinant *E.coli* aerobic growth processes. Therefore, the preparation and implementation of efficient and robust substrate supply profiles into bioreactors is the most important step in the implementation of efficient recombinant *E.coli* cultivation processes in industrial bioreactors. When the amount of substrate flow fed into the bioreactor is insufficient, the growth rate of recombinant *E.coli* bacteria is limited, and, respectively, at the same time, the productivity of the process is reduced. On the other hand, when the flow of the supplied substrate exceeds the maximum possible uptake of the substrate by *E.coli* bacteria, excess glucose, and metabolic by-products, mainly acetate, begin to accumulate in the cultivation medium. These products reduce the growth rate of *E.coli* bacteria and the rate of synthesis of the target product during the product formation phase.

Several solutions are known, disclosing various methods of forming and realizing a stream of a feeding substrate into a bioreactor.

The US patent US5595905A (granted 21.01.1997) discloses a control system for feeding a substrate into a bioreactor for fed-batch cultivation processes of bacteria or yeast. The control system is based on laboratory experiments of feeding substrate concentration measurements and calculation procedures that allow the calculation of the feeding substrate flow for the next stage of the culture experiment. The application of this control system to cultivation processes does not ensure the reproducibility of the processes in cases when at the beginning of the cultivation process the concentration of microorganisms and the initial growth rate of the culture differ slightly from the conditions of the standard cultivation process. Such a system is also inefficient if it is necessary to keep the rate of substrate consumption below the maximum during the synthesis phase of the target product. Also, such a system is difficult to implement in industrial cultivation processes because it requires high costs for frequent experimental measurements of substrate concentration in the bioreactor, and system maintenance requires additional labor costs for calibrating substrate measurement methods.

Another known method of supplying a feeding substrate is described in the US patents US6955892B2 (granted 18.10.2005) and US7521203B2 (granted 21.04.2009). Here, the flow of substrate is regulated by a regulator, according to the deviation from the pre-set and present concentration of dissolved oxygen, pO2, in the bioreactor medium, while adjusting the oxygen supply to the bioreactor to maintain a constant pH of the cultivation medium. When the cultivation medium begins to lack substrate, then the growth rate of microorganisms decreases, and the concentration of dissolved oxygen pO2 in the medium increases. The feeding substrate flow controller evaluates the deviation between the set and the actual pO2 value and adjusts the feeding substrate flow accordingly. The disadvantage of this method is that due to the strong interaction between the two control systems (pH and pO2), it is difficult to ensure the robustness of the systems to disturbances, and the proper quality and stability of the flow control of the supplied substrate. If the concentration of the substrate in the system increases due to various disturbances, the controllability properties of the *E.coli* cultivation process in the medium are impaired and the cultivation process becomes unstable.

One more method of supplying a feeding substrate flow to a bioreactor is described in the US patent US6284453B1 (granted 04.09.2001), where the substrate flow is adjusted to maintain a given growth rate of *E.coli* bacteria, which is determined by a series of direct and indirect measurements. This method cannot guarantee sufficient resiliency (robustness) and reproducibility of the processes. Due to typical disturbances during the cultivation process (substrate supply chain disturbances, disturbances due to anti-foam liquid supply, initial microorganism concentrations, and activity fluctuations), the growth-rate-only-control system cannot track integral values of the cultivation process variables, i.e., values of the concentrations of the biomass and target product, during the cultivation process and at the end of it. At the same time, this method cannot ensure the proper reproducibility of cultivation processes. Also, the implementation of this method requires performing measurements that can be difficult to perform in industrial bioreactors.

Akesson, M. (1999) [1] describes a method for regulating a feeding substrate based on test signals of the flow of feeding substrate to the bioreactor and the corresponding analysis of the dissolved oxygen sensor signal. The flow of substrate fed into the bioreactor is selected so that its increase can still cause an increase in bacterial growth and a corresponding decrease in the concentration of dissolved oxygen in the medium. By selecting the intensity of the feeding substrate flow, with the help of such test effects, the accumulation of excess substrate in the bioreactor is avoided. However, the implementation of this method during the cultivation process requires short-term, active test experiments to change the substrate flow fed to the bioreactor, and this limits the application of this method in real industrial processes.

An indirect method of regulating the feeding substrate is described by Kuprijanov A. et al. (2012) [2]. In this method, the flow of the feeding substrate is varied to maintain the given amount of carbon dioxide production profile during the cultivation process. However, the practical implementation of the mentioned method is quite complicated, because the implementation of regulation requires prior knowledge of carbon dioxide production and velocity profiles. In addition, the accuracy of this method is limited by the fact that the parameters of the regulators are not adapted during the cultivation of microorganisms and this causes large regulatory errors when the dynamics of the biotechnological process is changing.

One more indirect method of regulating the feeding substrate is described in the Lithuanian patent LT6395B (priority date: 24.07.2015), in which the flow of the substrate is changed to ensure the specific growth rate of microorganisms set by the production operator. This growth rate is determined indirectly by measuring the rate and amount of oxygen consumption of the culture grown in the bioreactor. The main disadvantage of this method is that when due to various disturbances, the concentration of the substrate in the bioreactor medium exceeds a certain limit, the cultivation process loses its controllability properties. Therefore, by increasing the substrate flow, it is no longer possible to achieve the set values of oxygen consumption rate and amount. As a result, the substrate further accumulates in the bioreactor, the cultivation process becomes unstable, and its productivity drops sharply.

The closest analogue to the present invention is a method of regulating the flow of a feeding substrate for fed-batch recombinant *E.coli* cultivation processes described in [3] V. Galvanauskas et al. (2018). In this method, robust feeding substrate profiles are calculated based on mathematical models of the cultivation process. The main disadvantage of this method is that the formation of feeding profiles requires the development of adequate mathematical models of the recombinant *E.coli* cultivation process for the developed processes. This requires high labor and time costs, therefore, the application of this method for industrial cultivation processes is limited.

The present invention is created to overcome the drawbacks or limitations observed in the solutions reviewed above. It aims to simplify and more stably regulate the flow of a feeding substrate into the bioreactor, ensuring better reproducibility of the cultivation process, and higher concentration and amount of target products synthesized.

SUMMARY OF THE INVENTION

The present invention discloses a method for developing and adapting robust substrate-feeding profiles. The method is based on the analysis of experimental data and provides better stability, reproducibility, and high productivity of recombinant *E.coli* cultivation processes for target protein synthesis. The method comprises the main four steps:

In the first step: experimental cultivation of recombinant *E.coli* is performed, during which the growth of bacteria is not limited and a high concentration of glucose (1-2 g/L) is maintained in the bioreactor medium.

In the second step: the obtained experimental bacterial glucose uptake data are processed by using a known smoothing cubic-spline technique, and the glucose uptake rate profile of the unlimited growth cultivation process is determined for, respectively: (i) *E.coli* bacteria growth phase and (ii) the target-product synthesis phase. Based on these results, further, there are planned robust growth-limiting and feeding substrate flow feed profiles into the bioreactor, for both phases of the recombinant *E.coli* cultivation process. Growth-limiting substrate feeding profiles allow to carry out stable and reproducible *E.coli* cultivation processes that are robust to various process disturbances and possible fluctuations of the initial biomass concentration and activity.

In the third step: *E.coli* restricted-growth cultivation experiments are performed with the previously formed limited *E.coli* growth substrate feeding profiles, and the substrate feeding profile is selected, the implementation of which allows to carry out a robust, highly-productive recombinant *E.coli* cultivation process. Such a cultivation process is defined as a reference cultivation process. Additionally, the profile of the process stability indicator is determined for this reference cultivation process, which is found by calculating the ratio of the amount of oxygen consumed to the amount of glucose supplied to the bioreactor during a selected cultivation time interval.

In the fourth step: industrial *E.coli* cultivation experiments are performed according to the established reference cultivation process and the formed reference substrate feeding profile. During them, the values of the above-mentioned cultivation process stability indicator are determined in real-time, they are compared with the values of the reference process indicator and the corresponding deviations are calculated. The increase in deviations indicates the possible undesirable accumulation of glucose concentration in the bioreactor medium. Based on the obtained deviation values, the process controller adapts the reference glucose feed profile in real-time.

This method of controlling the *E.coli* cultivation process is efficient and applicable for industrial *E.coli* cultivation processes/bioreactors because:

- it does not require specialized knowledge of process simulation and control theory from the production operator, as it is based on experimental data and uncomplicated measurements performed during the cultivation process;
- during the cultivation process, the feeding-substrate-flow profiles with high robustness are applied. They are adapted in an industrial bioreactor automatically and in real-time, if the self-regulating properties of the cultivation process can no longer compensate for the growth deviations of *E.coli* bacteria in the bioreactor due to high disturbances, and the cultivation process enters an unstable (uncontrollable) state;
- allows more simple and stable regulation of the substrate flow supplied into the bioreactor, at the same time, ensures better reproducibility of cultivation processes, higher concentrations, and amounts of the synthesized target products.

## DESCRIPTION OF DRAWINGS

The features and advantages of the invention are described in the detailed description of the invention with reference to the following drawings:

Fig. 1    illustrates the control method and its steps to form and adapt robust feeding profiles in the process of recombinant *E.coli* fed-batch cultivation.

Fig. 2    graphs showing variations of key process variables during the glucose non-limited fed-batch *E.coli* cultivation process (*Px* - fraction of the target protein in the cell, %; $0,01 \cdot Px \cdot X$ - concentration of the target protein in the cultivation medium, g/kg; $cumF_{gl}$ - glucose amount fed to the bioreactor, kg; X - biomass concentration in the medium, g/kg; *W* - the weight of the cultivation medium, kg; *OUR* - oxygen consumption rate, g/kg/h.

Fig. 3    glucose feeding profiles and different graphs of essential process variables corresponding to different levels of glucose consumption-rate limitation (85%, 90%, 95%, and unlimited).

Fig. 4    Graphs of the behaviour of important process variables using robust feeding profile for typical *E.coli* cultivation process, with disturbances ($X_d(0)=0,95 \cdot X(0)$, $\sigma_{max\_d} = 0,985_{\sigma max}$, $\sigma_d = 0,5\sigma$ in the short term), and without disturbances.

Fig. 5    deterioration of the cultivation process quality when maximum specific glucose consumption rate has decreased by 4%

Fig. 6    real-time adaptation of the reference feeding profile a) diagram of the bioreactor and PI controller with the feeding profile; b) estimate of oxygen uptake from glucose $Y_{OUR}$ / Fgl - the graph obtained by the 30-second sliding-window method.

Fig. 7    an example of a real-time adaptation of the feeding profile, and results in the recombinant *E.coli* cultivation process.

Fig. 8    Typical experimental results confirming the efficiency of the proposed method by testing of designed feeding profile in a real recombinant *E.coli* fed-batch cultivation process.

## DETAILED DESCRIPTION OF THE INVENTION

The details of the method of forming and adapting the feeding profiles are disclosed to provide a complete and comprehensible description of an exemplary embodiment of the invention. The details of specific examples of the implementation of the invention do not limit the implementation of the invention by other variants, which could be implemented without such specific instructions. Well-known procedures and ingredients have not been described in detail to avoid misleading examples of the invention. The specific example of the implementation of the method and the specific conditions do not limit the application of the method to other similar E.coli cultivation processes.

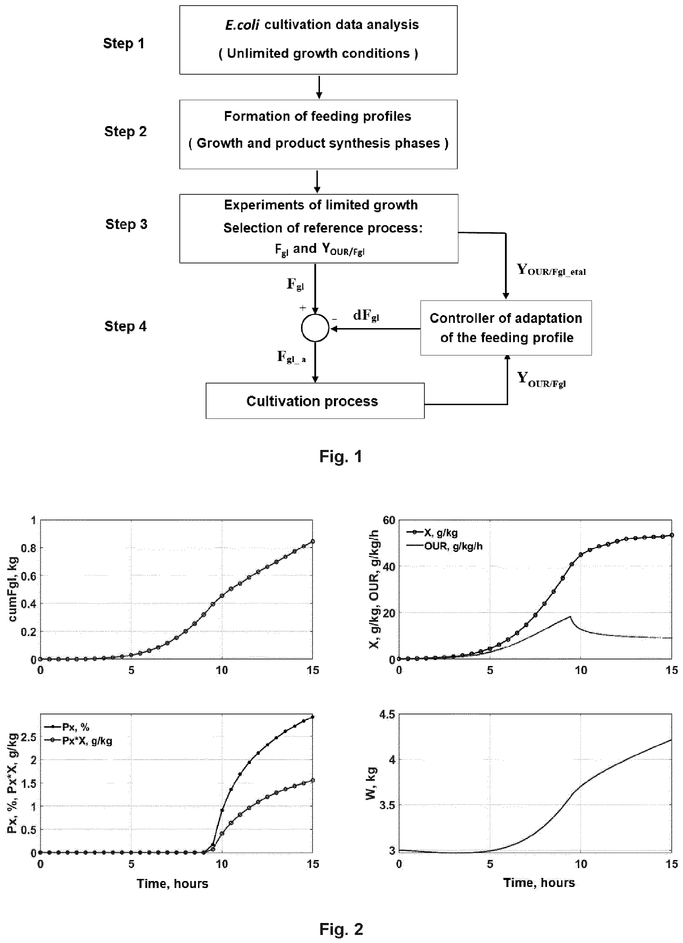

**Method steps.** The 4 essential steps of the method of forming and adapting robust feeding profiles for recombinant *E.coli* fed-batch cultivation processes are shown in Figure 1.

In the first step, an experimental fed-batch cultivation process of recombinant *E.coli* is performed. During it, manually or semi-automatically, a glucose concentration of 1-2 g/L is maintained in the cultivation medium, which ensures a rate of glucose uptake close to the maximum but does not allow the intensive formation of by-products of metabolism, which would inhibit the growth of *E.coli* bacteria. In this experimental process, the initial concentration of biomass and the induction schedule of the target product should be selected according to the *E.coli* cultivation protocol, normally used in the laboratory or plant. During the cultivation period, the amount of glucose consumed by *E.coli* bacteria is recorded with the discretion of 15-30 minutes. This variable reflects the maximum possible glucose uptake by *E.coli* throughout the cultivation period and is the basic variable needed for the further development of efficient and robust profiles of *E.coli* feeding with glucose.

In the second step, the maximum rate of glucose uptake by *E.coli* in the (i) *E.coli* growth phase and (ii) of the target-product formation phase is estimated, based on *E.coli* glucose uptake data under unlimited growth conditions and using the known smoothing cubic-spline technique. Based on these rate estimates, glucose supply profiles $F_{Gl}(t)$ to the bioreactor are prepared, optionally applying various possible levels of limiting glucose supply (and thus the growth rate of *E.coli* bacteria) (e.g., 85%, 90%, or 95%). The limited supply rate feeding profiles are formed by extending the duration of the cultivation process inversely proportionally to the selected limitation level (e.g., a ratio of 1/0.85) so that the total glucose consumption is the same as the glucose consumption in step (a). Recombinant *E.coli* cultivation processes comprise a biomass growth phase (i) and a target-product synthesis phase (ii). Using a robust feeding profile, the glucose flow into the bioreactor is planned to be 85-95% of the maximum glucose uptake rate recorded in step (a) (in the cultivation process under unlimited growth conditions). This limited glucose flow maintains a high rate of biomass growth, but at the same time maintains a relatively low glucose concentration in the bioreactor. Such a balanced feeding improves the controllability and reproducibility of the cultivation process and reduces the formation of metabolic by-products. When forming feeding profiles, the limited capabilities of oxygen transfer into the cultivation medium can be additionally taken into account, and, depending on this, the flow of the feeding glucose can be further reduced accordingly. To control the target-product synthesis phase (ii), *E.coli* growth limitation levels and, respectively, glucose feeding flow levels of 60-95% of the maximum glucose utilization rate (recorded under unlimited growth conditions) are selected in the feeding profiles.

In the third step, fed-batch cultivation experiments of recombinant *E.coli* are performed using the robust glucose feeding profiles $F_{Gl}(t)$ formed in the previous step (b) above. Based on the results of these experiments, a reference cultivation profile is selected that ensures the most suitable process robustness and a high amount of synthesized target product. According to the experimental data, the stability indicator $Y_{OUR/FGL\_etal}(t)$ is also calculated for this reference profile/cultivation process, which is the ratio of the amount of oxygen consumed to the amount of glucose supplied during a selected cultivation period.

In the fourth step, the selected reference robust feeding profile is realized in an industrial process of cultivating recombinant *E.coli*. The selected profile allows maintaining a productive, reproducible cultivation process even when the process is unpredictably affected by typical disturbances (short-term glucose supply disturbances, antifoam fluid pulses, initial 5-10% variations in *E.coli* bacteria concentrations and activity). However, if the amplitude of these disturbances is large enough, they can significantly reduce the maximum specific rate of glucose uptake in *E.coli* culture, and the cultivation process will become unstable. Such higher disturbances can still lead to uncontrolled accumulation of glucose in the bioreactor medium, the formation of metabolic by-products, and inhibit the synthesis of the target product. Therefore, this method provides a controller for adapting the reference feeding profile, which is activated in case of higher disturbances. In the controller, the values of the cultivation process stability indicator $Y_{OUR/FGL}(t)$ are checked in real-time, which then are compared with the values of the reference indicator $Y_{OUR/FGL\_etal}(t)$. A significant deviation of $Y_{OUR/FGL}(t)$ from the standard $Y_{OUR/FGL\_etal}(t)$ indicates undesired accumulation of glucose in the bioreactor. The adaptation controller is a typical proportional-integrating *PI* controller that responds to the deviations of the $Y_{OUR/FGL\_etal}(t)$ indicator and forms a corrective feed flow $dF_{Gl}(t)$, and the *PI* controller-corrected result - adapted feed flow $F_{Gl\_a}(t)$ - is supplied to the bioreactor. The profile adaptation function extends the possibilities of the cultivation process control method. It allows applying the selected reference feeding profile even then when due to strong disturbances, the reference feeding profile itself can no longer maintain stability, high efficiency, and reproducibility of the cultivation process.

The influence of temperature and other parameters determining the *E.coli* cultivation and bioreactor processes is considered insignificant and solved in the present invention. Modern bioreactors have reliable T, pH, pO2 control systems, which are not detailed in the description, and are considered to be sufficiently and properly functioning.

**The environment of method and profiles testing.** The developed method, feeding profiles, and their efficiency were tested in a "virtual bioreactor" (VBR). The function of this VBR is to simulate a real *E.coli* cultivation process in which the Interferon-alpha-5 (IFNa5) protein is synthesized in recombinant *E.coli* bacteria [4,5]. The basis of VBR is a mathematical model of the growth process of recombinant *E.coli*. It consists of the mass-balance equations of the main modeled components and specific reaction rate expressions describing the biochemical conversion rates. The main parameters of VBR status are biomass concentration X, glucose concentration S, target protein IFNa5 concentration (percentage in a cell, insoluble inclusion bodies) Px, culture medium weight W:

$$\frac{dX}{dt} = \mu \cdot X - \frac{F}{W} \cdot X \qquad (1)$$

$$\frac{dS}{dt} = -\sigma \cdot X - \frac{F}{W} \cdot S + \frac{F_S}{W} \cdot S_F \qquad (2)$$

$$\frac{dP_X}{dt} = Ind \cdot K_P \cdot \mu \qquad (3)$$

$$\frac{dW}{dt} = F - F_{smp} \qquad (4)$$

The total mass flow in the bioreactor is calculated by taking into account sampling $F_{smp}$, substrate supply $F_S$, acidity *pH* control alkaline buffer $F_b$, medium weight loss by removing $CO_2$ from the exhaust gas $F_{CO2}$, and evaporation of water through the ventilation duct $F_e$. In the VBR applications, the following flows are modeled according to empirical dependences, taking into account the dynamics of biomass growth and other important variables of the biotechnological process:

$$F_b = Y_{XB} \cdot \mu \cdot X \cdot W \qquad (5)$$

$$F_{CO2} = -R \cdot OUR_W \qquad (6)$$

$$F_e = -k_e \cdot W \qquad (7)$$

$$F = F_S + F_b + F_{CO2} + F_e \qquad (8)$$

In addition, the rate of oxygen uptake OUR (per weight unit, and for the total mass of growth medium) is calculated by relating the two components in which oxygen is consumed for (i) growing the biomass and (ii) maintaining the biomass:

$$OUR = Y_{OX} \cdot \mu \cdot X + m_{OX} \cdot X \qquad (9)$$

$$OUR_W = OUR \cdot W. \qquad (10)$$

The specific reaction rates of the main components, glucose S and biomass X, are simulated using kinetic relationships reflecting the rate-limiting and inhibition of the reactions. The specific rate of glucose uptake $\sigma$ is conditioned by the substrate limitation, and inhibitions due to the high concentration of biomass and the concentration of the target-protein synthesized in intracellular insoluble inclusion bodies:

$$\sigma = \sigma_{max} \frac{S}{K_S + S} \cdot \frac{K_X}{K_X + X} \cdot \frac{K_{Px}}{K_{Px} + P_X} \qquad (11)$$

The specific growth rate of biomass $\mu$ is simulated taking into account the consumption of the substrate for growth and maintenance of viability, m. At high glucose concentrations, recombinant *E.coli* releases acetates and other by-products of excess metabolism. This reduces the biomass yield and the resulting by-products can inhibit the rate of glucose uptake. In the cultivation process studied, the inhibition caused by these by-products was insignificant, so the specific growth rate of biomass is modeled using the following simplified equation:

$$\mu = (\sigma - m) \cdot Y_{XS} \cdot \frac{K_{YS}}{K_{YS}+S} \qquad\qquad (12)$$

In an experimental cultivation process, the formation of the target protein IFNa5 was initiated by the addition of a rational amount of inducer (IPTG) when the biomass concentration X during cultivation exceeded 40 g/kg (*Ind=0*, until *X<40 g/kg*, and *Ind=1* after *X≥40g/kg*). The target protein is formed in the insoluble inclusion bodies formed in *E.coli* cells, and the rate of formation, in this case, was proportional to the specific growth rate. The parameters of the model equations (1-12) were determined from experimental data from 15 fed-batch recombinant E.coli cultivation processes performed in a 7 L bioreactor (working volume 3 L) [5]. The developed model adequately describes the process of cultivating recombinant *E.coli,* and the quality of the model is sufficient to test the methodology of feeding profiles formation and adaptation developed by the present invention. The developed model is implemented with a user-friendly interface of VBR, which allows simulating various feeding scenarios and typical disturbances of the cultivation process, as well as measurement noises. Next, the VBR model was used to test the efficiency of the feeding profiles formation and adaptation procedure.

**Data analysis and forming the feeding profiles.** According to the method steps, in the first step, under unlimited growth conditions, a cultivation experiment is performed with typical initial conditions, to determine the maximum glucose uptake capacity of the cells. The initial graph of biomass concentration and culture induction was consistent with a typical *E.coli* growth cycle. The above-mentioned VBR model was used for the experiment, in which the glucose concentration S was manually controlled in the range of 1-1.5 g/kg, the initial biomass concentration was *X(0)=0.15 g/kg*, and the induction was started when the biomass concentration X exceeded 40 g/kg. During cultivation, the amount of pure glucose *cumFgl* fed to the bioreactor is recorded every half hour, and the biomass concentration and the concentration of the target product are measured. In addition, another important parameter is recorded in real-time during the cultivation - the oxygen uptake rate OUR. The change in these essential variables over the cultivation period (under unlimited glucose conditions) is shown in Figure 2. The obtained glucose consumption data *cumFgl* are used to estimate the rate of glucose consumption using the smoothing cubic-spline method. Based on the maximum glucose uptake rate, different feed rate scenarios (profiles) were developed, with different levels of glucose uptake rate/supply limitation in (i) the biomass growth phase and (ii) the target protein synthesis phase. Glucose feeding begins when the initial glucose in the bioreactor has been consumed. In all feeding scenarios, the total amount of glucose fed to the bioreactor is the same. Therefore, cultivation processes/experiments with a limited rate of glucose uptake extend over time and are correspondingly longer.

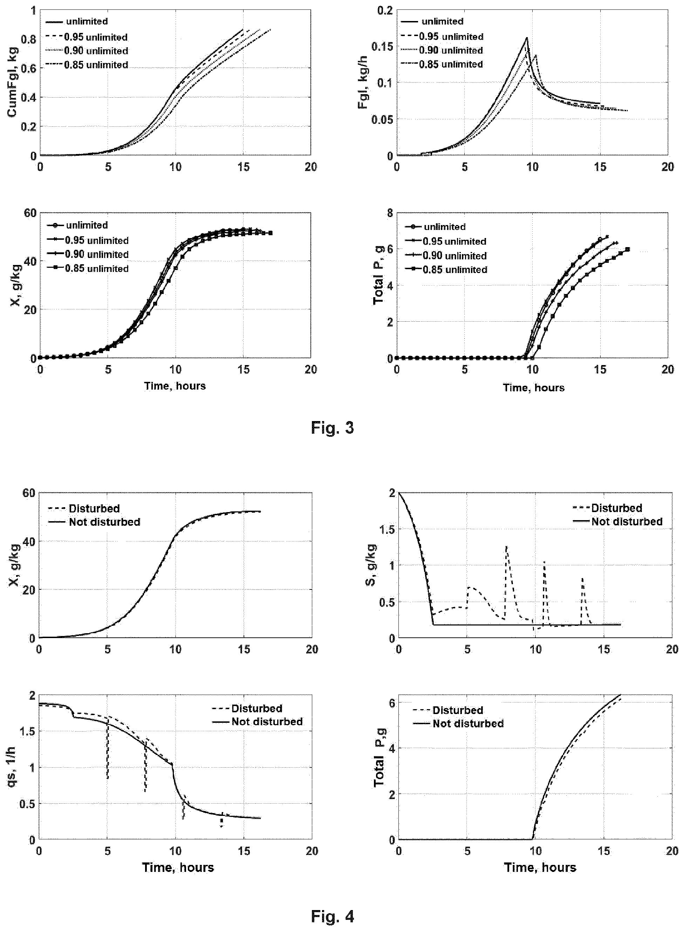

The planned cultivation experiments were performed in a "virtual bioreactor". Their results, with different feeding profiles and limitation levels, are shown in Figure 3. Based on these results, an efficient feeding profile can be selected.

**Selection of effective profiles.** Analysis of VBR data from cultivation experiments provides a possibility to assess the process quality for a variety of glucose feeding profiles. It is important to note that in the cases studied, the target protein is formed in *E.coli* cells in the form of insoluble inclusion bodies, and the rate of protein synthesis is proportional to the specific growth rate. Therefore, high glucose uptake rates are preferred for efficient protein production. On the other hand, excessive consumption rate increases the formation of metabolic by-products, and the growth rate decreases. As shown in the results in Figure 3, a culture experiment with low glucose limitation (feed rate is 0.95 of the unlimited feed) allows achieving the most efficient process and the highest amount of target protein produced. However, to ensure greater reproducibility and stability of the process, it is useful to choose a feeding profile that covers only 90% of the unlimited feeding. In this case, the amount of target protein obtained at the end of cultivation is 6.3 g and is about 3% lower than that obtained in the case of unlimited feeding, but the cultivation process becomes more robust to various disturbances. Therefore, this feeding profile was chosen as the reference profile for further studies.

**Testing the feeding profile robustness.** The robustness of the selected reference feeding profile to disturbances was tested by creating typical disturbances during the cultivation process. Simulation experiments show that the reference glucose feeding profile ensures an efficient and stable *E.coli* cultivation process with an initial biomass concentration within ±15% of the typical value. Also, the cultivation process remains efficient and stable, simulating a short-term (5 min) decrease in the specific rate of glucose uptake (up to 50%). Such disturbances are typical in real cultivation processes, especially when antifoaming fluid is injected into the bioreactor. This action can significantly reduce glucose consumption and biomass growth rate in the short term, but due to the resilience (robustness) of the feeding profile, it does not significantly affect the cultivation process. Using the reference profile, the cultivation process remains stable until the maximum specific glucose consumption $\sigma_{max}$ decreases by more than 3%. The variation of the essential variables in a typical recombinant *E.coli* cultivation process, with and without typical disturbances, is shown in Figure 4. As can be seen, in the case of relatively significant disturbances, the target-protein content is only 2.9% lower than that obtained in the disturbance-free process.

The simulation results show that the feeding profiles formed by the method of the present invention (induction is started when the glucose consumption *cumFgl* exceeds 0.425 kg) ensure a stable *E.coli* cultivation process with self-regulation. Therefore, small disturbances in real cultivation processes, especially deviations in the initial biomass concentration or short-term disturbances in the specific glucose consumption rate, as well as small changes in the specific maximum substrate consumption rate, do not significantly change the quality of the cultivation process: the produced biomass and target protein, are as in the case of an undisturbed process. Consequently, it is not necessary to adapt the feeding profile in the presence of minor disturbances to the cultivation process. This is especially important in industrial cultivation processes, where for enabling any additional process correction or stabilization actions, there are required complex changes in rules and recipes of the cultivation process.

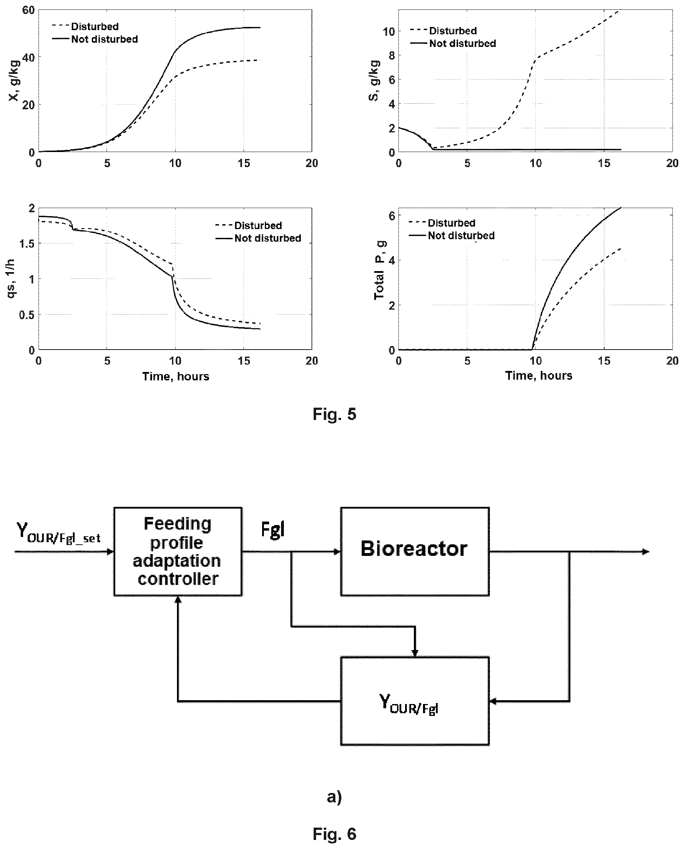

**Adaptation of feeding profile in the cultivation process.** However, industrial cultivation processes can also experience major disturbances, in which case the reference feed profile is no longer able to maintain process stability, which can destabilize the process and make it inefficient due to the high glucose amount accumulated in the bioreactor. A typical experimental example is shown in Figure 5. It simulated that due to certain changes in *E.coli* bacteria, the maximum specific rate of glucose uptake was reduced by 4% during the cultivation. This resulted in glucose accumulation, deteriorated process quality, and a 35% reduction in the amount of the target protein produced.

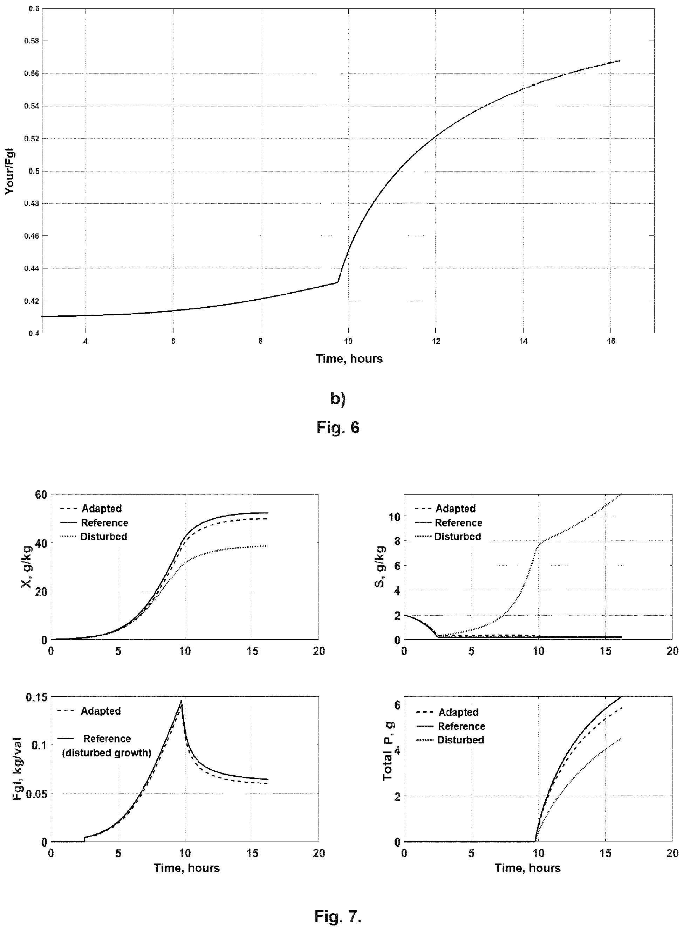

In such cases, a step of controlling the cultivation process is provided to indirectly monitor the accumulation of glucose in the cultivation medium and to adapt the reference glucose feeding profile in real-time. For this purpose, real-time estimation of the ratio of actual oxygen consumption to the supplied glucose is performed using the sliding window method (30 sec. window) and compared with the reference indicator $Y_{OUR/Fgl\_set}$, whose temporal profile is shown in Figure 6 (b). The velocity form proportional-integrating *PI* controller is used to adapt the reference measurement profile. The real-time adaptation profile of the reference power profile is shown in Figure 6 (a). This step of adapting the reference profile allowed the elimination of excess glucose accumulation in the bioreactor medium and improved the production efficiency of the target protein.

The results of profile adaptation are shown in Figure 7. As can be seen from the graph, the profile adaptation step helped to prevent excessive accumulation of glucose in the cultivated medium and improved the quality of the process. The amount of 5.9 g of the target protein produced is close to the amount of 6.3 g of protein obtained in the initial standard cultivation process. Meanwhile, in a cultivation process in which glucose accumulation was not controlled, the amount of target protein produced was only 4.3 g.

**Applications.** The method of developing and adapting robust glucose feeding profiles disclosed by the present invention enables the realization of stable, reproducible, and efficient recombinant *E.coli* fed-batch cultivation processes, during which biopharmaceutical target products for various purposes can be synthesized.

The description of the invention lists a number of characteristics and advantages of the method, the details of which can be changed without departing from the principles of the invention, in accordance with the most widely understood meanings of the terms used in the claims.

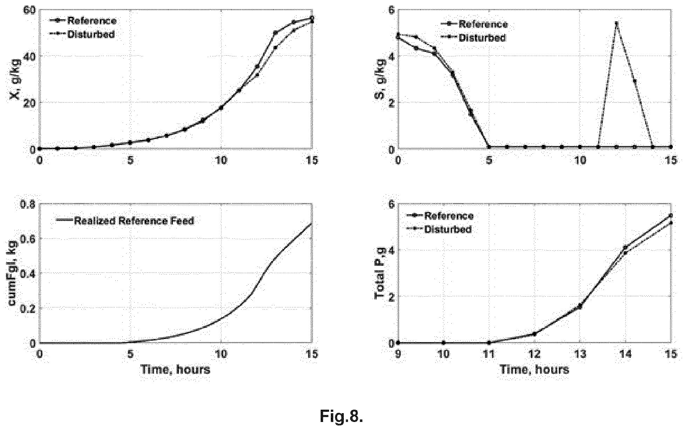

**Experimental results in real bioreactor and cultivation process.** The proposed method was also validated in a real bioreactor using recombinant *E.coli* bacteria (BL21 - DE3 strain), for producing a recombinant protein - interferon alpha 5 (IFNa5 in form of inclusion bodies). Fed-batch cultivations were performed in a 7 L bioreactor (working volume 3 L), the reference feeding profile was designed following the steps proposed in the invention. To achieve the most efficient IFNa5 production and a stable cultivation process, the glucose uptake rate limitation in growth and product formation phases was set to 90% of the maximum glucose uptake rate. The designed reference substrate feeding profile was used in various real process validation tests, where sufficient process reproducibility and robustness to process disturbances were achieved. The validation results were similar to those obtained in the experiments with the virtual bioreactor. A typical real cultivation experiment that demonstrates the robustness of the designed substrate feeding profile is shown in Figure 8. In this experiment, after the addition of inducer (IPTG) to the cultivation media, an untypical decrease in glucose consumption rate was observed and glucose concentration *S* rapidly began to accumulate in the media. However, due to the robustness of the designed feeding profile, after two hours the effect of this disturbance was compensated and the amount of target protein *P* at the end of cultivation was only 5% lower than in the reference process. It is important to mention that if the reference feeding profile had been formed according to the maximum glucose uptake rate, the cultivation process would have become unstable and unproductive.

The previous paragraph is added, to describe experimental results presented in Figure 8, and to prove that these experimental results only validate the previous simulation results in the virtual reactor (VBR) and are comprised in the concept and scope of the described and claimed invention.

LIST OF NON-PATENT CITATIONS

[1] Akesson, M. (1999, "Probing control of glucose feeding in Escherichia coli cultivations", ISRN LUTFD2/TFRT-1057-SE. PhD thesis, Department of automatic control, Lund Institute of Technology, Sweden).

[2] Kuprijanov A., Schaepe S., Aehle M., Simutis R., Lübbert A., "Improving cultivation processes for recombinant protein production" // Bioprocess and Biosystems Engineering. Berlin: Springer. ISSN 1615-7591. 2012, Vol. 35, iss. 3, p. 333-340).

[3] V. Galvanauskas, R. Simutis, D. Levišauskas, M. Butkus and R. Urniezius, "Development and Investigation of Efficient Substrate Feeding and Dissolved Oxygen Control Algorithms for Design of Recombinant E. coli Cultivation Process", 2018 IEEE 14th International Conference on Automation Science and Engineering (CASE), Munich, 2018, pp. 657-660, doi: 10.1109/COASE.2018.8560495).

[4] V.A. Bumelis, "Method for producing interferon-alpha 5," European Patent No. EP2532734A1, Jan 31, 2011.

[5] R. Urniezius, A. Survyla, D. Paulauskas, V.A. Bumelis, and V. Galvanauskas, "Generic Estimator of Biomass Concentration for Escherichia coli and Saccharomyces cerevisiae Fed-Batch Cultures Based on Cumulative Oxygen Consumption Rate," Microbial Cell Factories, vol. 18, 190, Nov. 2019.

**Claims**

1. A method for controlling a fed-batch cultivation process of recombinant *E.coli* bacteria in a bioreactor, **characterized in that** it is based on the formation and adaptation of experimental *E.coli* feeding profiles, comprising at least the steps:

   a. experimental evaluating of the maximum possible glucose uptake by *E.coli* bacteria under conditions of unlimited growth;
   b. from the recorded glucose uptake data, estimating the maximum rate of glucose uptake during the *E.coli* cultivation process and prepare process-robust glucose feeding profiles $F_{Gl}(t)$ to the bioreactor;
   c. $F_{Gl}(t)$ profiles are tested experimentally and a reference profile is selected, based on the robustness of the cultivation process and the amount of target-product, and cultivation process stability indicator $Y_{OUR/FGL\_etal}(t)$ is calculated which is the ratio of oxygen consumption to glucose uptake over a selected time interval.
   b) The reference feeding profile is implemented in the *E.coli* cultivation process using the profile adaptation controller **PI** to adjust the glucose flow $F_{Gl\_a}(t)$ according to the difference between the actual cultivation process stability indicator $Y_{OUR/FGL}(t)$ and the reference process indicator profile $Y_{OUR/FGL\_etal}(t)$.

2. The method according to claim 1, **wherein** in the first step (a), a fed-batch cultivation experiment of recombinant *E.coli* is performed, controlling the glucose concentration in the cultivation medium to ensure a rate of maximum glucose uptake but avoid intense formation of metabolic by-products inhibiting bacterial growth and synthesis of the target-product, and the amount of glucose consumed during cultivation is recorded, which is considered to be the maximum possible amount of glucose uptake by *E.coli* during the cultivation process.

3. The method according to claim 1, **wherein** in the second step (b), based on the experimental data collected on the amount of glucose consumed during cultivation and using the smoothing cubic-spline technique, the maximum rate of glucose uptake is estimated (i) in the *E.coli* bacteria growth phase and (ii) ) in the production of the target-product phase, and based on these glucose utilization rate estimates, glucose flow delivery profiles to the bioreactor are prepared with glucose uptake rate limiting levels in the *E.coli* cultivation process.

4. The method according to claim 1, **wherein** in the third step (c), fed-batch cultivation experiments with recombinant *E.coli* are performed by realizing the planned robust glucose delivery profiles $F_{Gl}(t)$ and the results are used to select a reference cultivation process that provides adequate robustness of the process and the high amount of target-product, as well as the $Y_{OUR/FGL\_etal}(t)$ profile of the cultivation process stability indicator calculated from the experimental data for the reference process, which is found by calculating the ratio of oxygen consumption during cultivation to the amount of glucose supplied to the bioreactor over a selected time interval.

5. The method according to claim 1, **wherein** in the fourth step (d), the reference profile is implemented in an industrial recombinant *E.coli* cultivation process, and the reference glucose feeding profile adaptation controller **PI** is activated in the event of significant unpredictable disturbances that may cause process instability:

   • records in real-time the value of the cultivation process stability indicator $Y_{OUR/FGL\_etal}(t)$, which is the ratio of the amount of oxygen consumed to the amount of glucose supplied,
   • compares $Y_{OUR/FGL}(t)$ with the values of the reference process indicator $Y_{OUR/FGL\_etal}(t)$,

• adapts the reference glucose feeding flow $F_{Gl\_a}(t)$ supplied to the bioreactor according to the obtained difference deviation.

6. The method according to claims 1 and 3, **characterized in that** in (i) the *E.coli* bacteria growth phase, the limitation for glucose uptake rate by *E.coli* is set within the range of 85-95% of the maximum glucose uptake rate.

7. The method according to claims 1 and 3, **characterized in that** in (ii) the target-product synthesis phase, the limitation for glucose uptake rate by *E.coli* is set within the range of 60-95% of the maximum glucose uptake rate.

8. The method according to claims 1, 3, 6-7, **characterized in that** for the process under study (i) in the *E.coli* bacteria growth phase and (ii) in the protein IFN$\alpha$5 as the target-product synthesis phase, to achieve the most efficient production, the level of glucose uptake rate limitation is set to 95% of the maximum glucose uptake rate.

9. The method according to claims 1, 3, 6-8, **characterized in that** for the process under study (i) in the E. coli bacteria growth phase and (ii) in the protein IFN$\alpha$5 as the target-product synthesis phase, to achieve the most efficient production and a stable cultivation process, the preferred level of glucose uptake rate limitation is set to 90% of the maximum glucose uptake rate.

10. The method according to claims 1 and 5, **characterized in that** the reference glucose feeding profile adaptation controller PI is the velocity form of proportional-integral controller.

Fig. 1

Fig. 2

Fig. 3

Fig. 4

Fig. 5

a)

Fig. 6

b)

Fig. 6

Fig. 7.

Fig.8.

Europäisches
Patentamt

European
Patent Office

Office européen
des brevets

# EUROPEAN SEARCH REPORT

**Application Number**

EP 22 16 4604

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | LIN H Y ET AL: "Determination of the maximum specific uptake capacities for glucose and oxygen in glucose-limited fed-batch cultivations of Escherichia coli", BIOTECHNOLOGY AND BIOENGINEERING, JOHN WILEY, HOBOKEN, USA, vol. 73, no. 5, 16 April 2001 (2001-04-16) , pages 347-357, XP071032780, ISSN: 0006-3592, DOI: 10.1002/BIT.1068 * the whole document * | 1-10 | INV. C12M1/34 C12M1/36 C12N1/20 C07K14/195 |
| A,D | GALVANAUSKAS VYTAUTAS ET AL: "Development and Investigation of Efficient Substrate Feeding and Dissolved Oxygen Control Algorithms for Design of Recombinant E. coli Cultivation Process", 2018 IEEE 14TH INTERNATIONAL CONFERENCE ON AUTOMATION SCIENCE AND ENGINEERING (CASE), IEEE, 20 August 2018 (2018-08-20), pages 657-660, XP033463237, DOI: 10.1109/COASE.2018.8560495 [retrieved on 2018-12-04] * the whole document * | 1-10 | |
| A | STEFAN GNOTH ET AL: "Control of cultivation processes for recombinant protein production: a review", BIOPROCESS AND BIOSYSTEMS ENGINEERING, SPRINGER, BERLIN, DE, vol. 31, no. 1, 5 October 2007 (2007-10-05), pages 21-39, XP019564177, ISSN: 1615-7605 * the whole document * | 1-10 | TECHNICAL FIELDS SEARCHED (IPC) C12M C12R C12N C07K |

-/--

The present search report has been drawn up for all claims

| Place of search | Date of completion of the search | Examiner |
|---|---|---|
| Berlin | 28 September 2022 | Cubas Alcaraz, Jose |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 22 16 4604

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | EMMANUEL ANANE ET AL: "A model-based framework for parallel scale-down fed-batch cultivations in mini-bioreactors for accelerated phenotyping", BIOTECHNOLOGY AND BIOENGINEERING, JOHN WILEY, HOBOKEN, USA, vol. 116, no. 11, 30 July 2019 (2019-07-30), pages 2906-2918, XP071131681, ISSN: 0006-3592, DOI: 10.1002/BIT.27116 * the whole document * ----- | 1-10 | |

TECHNICAL FIELDS
SEARCHED (IPC)

The present search report has been drawn up for all claims

| Place of search | Date of completion of the search | Examiner |
|---|---|---|
| Berlin | 28 September 2022 | Cubas Alcaraz, Jose |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5595905 A
- US 6955892 B2
- US 7521203 B2
- US 6284453 B1
- LT 6395 B
- EP 2532734 A1, V.A. Bumelis

**Non-patent literature cited in the description**

- Probing control of glucose feeding in Escherichia coli cultivations. **AKESSON, M.** ISRN LUTFD2/TFRT-1057-SE. PhD thesis. Department of automatic control, Lund Institute of Technology, 1999
- Improving cultivation processes for recombinant protein production. **KUPRIJANOV A. ; SCHAEPE S. ; AEHLE M. ; SIMUTIS R. ; LÜBBERT A.** Bioprocess and Biosystems Engineering. Springer, 2012, vol. 35, 333-340
- **V. GALVANAUSKAS ; R. SIMUTIS ; D. LEVIŠAUSKAS ; M. BUTKUS ; R. URNIEŽIUS.** Development and Investigation of Efficient Substrate Feeding and Dissolved Oxygen Control Algorithms for Design of Recombinant E. coli Cultivation Process. *2018 IEEE 14th International Conference on Automation Science and Engineering (CASE), Munich,* 2018, 657-660
- **R. URNIEZIUS ; A. SURVYLA ; D. PAULAUSKAS ; V.A. BUMELIS ; V. GALVANAUSKAS.** Generic Estimator of Biomass Concentration for Escherichia coli and Saccharomyces cerevisiae Fed-Batch Cultures Based on Cumulative Oxygen Consumption Rate. *Microbial Cell Factories,* November 2019, vol. 18, 190